# EUROPEAN PATENT SPECIFICATION

(11) **EP 1 206 225 B1**
(45) Date of publication and mention of the grant of the patent: **09.03.2005**
(21) Application number: 00953086.6
(22) Date of filing: 25.07.2000
(51) Int. Cl.: A61F 2/30, A61F 2/28, A61P 19/00

(54) **IN VITRO REPAIR OF BONE AND/OR CARTILAGE DEFECTS**
IN VITRO HEILUNG VON KNOCHEN- UND/ODER KNORPELSCHÄDEN
REPARATION IN VIVO DE DEFAUTS OSSEUX ET/OU DU CARTILAGE

(30) Priority: 28.07.1999 SE 9902807; 28.07.1999 SE 9902808
(43) Date of publication of application: 22.05.2002
(60) Divisional application: 04002205.5
(73) Proprietor: Interface Biotech A/S, 2600 Glostrup (DK)
(72) Inventor: OSTHER, Kurt, Scottsdale, AZ 85259 (US); STORGAARD, Peter, DK-3390 Hundested (DK)
(74) Representative: Johansen, Marianne
(86) International application number: PCT/EP2000/007111
(87) International publication number: WO 2001/006949

(56) References cited:
- EP-A- 0 530 804
- EP-A- 0 934 750
- WO-A-96/24310
- WO-A-98/00183
- WO-A-98/30234
- WO-A-98/33515
- DE-A- 19 520 867
- DE-A- 19 648 876
- US-A- 4 846 835
- US-A- 5 759 190
- US-A- 5 876 452
- CASTRO-MALASPINA H ET AL: "CHARACTERIZATION OF HUMAN BONE MARROW FIBROBLAST COLONY-FORMING CELLS (CFU-F) AND THEIR PROGENY" BLOOD,W.B. SAUNDERS, PHILADELPHIA, VA,US, vol. 56, no. 2, August 1980 (1980-08), pages 289-301, XP000940570 ISSN: 0006-4971
- PITTENGER M F ET AL: "HUMAN MESENCHYMAL STEM CELLS CAN BE DIRECTED INTO CHONDROCYTES, ADIPOCYTES AND OSTEOCYTES" MOLECULAR BIOLOGY OF THE CELL,US,BETHESDA, MD, vol. 7, 7 December 1996 (1996-12-07), page 305A XP000882095 ISSN: 1059-1524
- PITTENGER M F ET AL: "CHARACTERIZATION OF THE DIFFERENTIATION OF HUMAN MESENCHYMAL STEM CELLS TO THE CHONDROGENIC, ADIPOGENIC AND OSTEOGENIC PATHWAYS" MOLECULAR BIOLOGY OF THE CELL,US,BETHESDA, MD, vol. 8, November 1997 (1997-11), page 112A XP000882127 ISSN: 1059-1524

## Description

### Field of invention

The present invention relates to method and materials for in vivo repair of cartilage defects or bone and cartilage defects in joints in mammals, such as human and horses.

### Background of invention

More than one million human arthroscopic procedures and total joint replacements are performed each year in the U.S. and Europe together. Included in these numbers are in the U.S., about 90,000 total knee replacements, and around 50,000 procedures for repairing defects in the knee alone per year (In: Praemer, A., Fumer, S., Rice, D.P., Musculoskeletal Conditions in the United States, Park Ridge, III.: American Academy of Orthopaedic Surgeons, 1992, 125).

Among different breeds of horses in U.S. and in Europe there are around five million registered "expensive" Thoroughbred Racehorses used in horse races, whereof an estimated 60% is directly or indirectly owned by U.S. horse-owners. Of all breeds Thoroughbreds is the most frequent sufferer of degenerative joint disease, mostly in the form of osteochondritis dissecans (OCD). The most often joint affected is the so-called stifle joint (femoropatellar joint, hind leg). The most common age for horses and especially racehorses to develop OCD is between 1 and 6 years old. The earlier, the training of thoroughbreds is started (1 year old or less) the more frequent the disorder will appear.

In general surgery including arthroscopic intervention is used in most of the cases with clinical symptoms. Around 64% of the horses treated return to their previous use (racing, etc.). Approximately 35 % of the horses cannot return to their previous use within racing or have fewer possibilities of obtaining previous levels of racing.

A second condition that can be observed, described as OCD, is fragmentation at the back of the fetlock off the proximal or plantar aspect of the first phalanx or long pastern bone. A third, often trauma related condition of racehorses is cartilage damage to the cannon bone condyles, which actually is not a true OCD. OCD in shoulder joints often affects large areas of the joint surface and secondary osteoarthritis is common.

Conservative or medicinal treatment has only a limited effect and is mainly targeted at keeping the horse free from pain. The drugs are typically non-steroidal anti-inflammatory drugs (NSAID). Surgery of the shoulder is difficult due to the depth of the joint below the muscles in the area.

The effect on equine articular cartilage repair in joints medicated with polysulfated glycosaminoglycan (PSGAG) or sodium hyaluronate (SH) has been tested at Ohio State University, College of Veterinary Medicine, Columbus, Ohio. Results of this study indicated no beneficial effect on clinical parameters, the gross appearance, or the microscopic appearance of defects when compared to saline controls. Some evidence from this study suggests that intraarticular PSGAG may have a detrimental effect on the healing of articular cartilage.

Occurrence of subchondral cystic lesions, also called bone cysts or osseous cystlike lesions are commonly recognised abnormalities of bones and joints that may or may not cause lameness. The most troublesome cysts are articular cysts. Controversy exists as to whether these lesions are a manifestation osteochondritis secondary to a joint trauma, or a combination of stress and trauma.

Human articular cartilage is incapable of undergoing self-repair since chondrocytes lose their mitotic ability during the first year of life. Defects in articular cartilage, especially in weight-bearing joints, will predictably deteriorate toward osteoarthritis. No conventional method may prevent this deterioration.

Drilling of the subchondral bone can lead to fibrocartilage formation, which is non-resilient and can only be considered a temporary repair that slowly degrades. Animal studies have indicated that introducing proliferating chondrocytes such as articular chondrocytes may reliably reconstruct joint defects (Robinson D., et al., lsr. Med. Assoc.J., 2000, 2:290).

Autologous chondrocyte implantation (ACI) has proven clinically effective in restoring hyaline-like cartilage to isolated pathological full thickness chondral lesions of the human knee. Several authors have performed chondrocyte implantation in humans with excellent results (Brittberg et al., 1994 New Engl. J. Med.; Minas, T., Am. J. Orthop. 1998, 11:739).

A method for regeneration treatment of cartilage would be an advantage and could be performed at an earlier stage of a joint damage, reducing the number of humans needing artificial joint replacement surgery. Previously, cleaning or resurfacing the cartilage structure have been attempted using subchondral drilling, abrasion, etc. whereby diseased cartilage and even subchondral bone is excised (Insalt, J., Clin. Orthop. 1974,101:61; Ficat, R.P., et al., Clin Orthop. 1979, 144:74; Johnson, L.L., In: (McGinty, J.B., Ed.) Operative Arthroscopy, New York, Raven Press, 1991, 341).

Other methods such as suturing a periosteal flap (for instance removed from tibia) over the defect has been used either as a treatment procedure in itself, or has been used in combination with implantation of cultured (e.g., autologous) chondrocytes. The methods using this combination have in principal been developed by Brittberg et al., (Brittberg, M., et al., New Engl. J. Med., 1994,331:889).

Cells cultured using the methods described by Brittberg et al., Engl. J. Med., 1994, 331:889 are used for autologous implantation into knee joints of patients. Kurt Osther et al. have described, in U.S. patent No. 5,759,190, the use of a barrier towards bone that may be useable in osteoarthritis type of lesions in theory, in order to prevent bleeding and proliferation of stem cells from the underlying denuded cancellous bone. The barrier is made of collagen type I.

The major role in the success of an autologous chondrocyte implantation is profoundly dependent on the condition of the chondrocytes to be implanted. It is of major importance that the chondrocyte culture is viable and inducible for both proliferation and, when implanted, capable of providing a sufficient matrix production (Mayhew, T.A., Tissue Engl. 1998, 4:325). It is important that the chondrocytes to be implanted are cultured under the most gentle and strictest culture methods, avoiding unnecessary enzymatic damage of the cell membrane, and at the same time obtaining the most optimal chondrocyte culture as possible to produce a healthy hyaline articular cartilage. The use of the cell culturing method, which is most gentle towards the chondrocyte is of utmost importance in order to obtain a sufficiently healthy implant cartilage, capable of interacting with the surrounding cartilage *in situ.* Even equally important is to create an optimal cartilage structure as part of the repair of osteoarthritic defects.

The invention provides improved membranes to be used for the treatment of cartilage or cartilage and bone defects in mammals. The inventions also provide new methods for the treatment of cartilage or bone and cartilage defects in mammals.

### Summary of the invention

This application discloses method and materials for *in vivo* repair of cartilage defects and bone and cartilage defects in joints in mammals, such as human and horses.

Accordingly, in a first aspect the invention relates to a cartilage membrane having at least one surface part carrying a composition comprising at least one stimulation molecule which is capable of inducing a signal transduction in chondroblasts/chondrocytes resulting in the chondroblasts/chondrocytes producing and secreting matrix components which form hyalin cartilage or more specifically hyalin articular cartilage.

In another aspect the invention relates to an interface membrane with a first surface part and a second surface part both carrying a composition comprising at least one stimulation molecule which is capable of inducing a signal transduction in chondroblasts/chondrocytes and osteoblasts/osteocytes.

In yet another aspect the invention relates to a method for in vivo repair of cartilage defects in joints in mammals, comprising applying, over a cartilage free cavity of a joint, a cartilage membrane with a first surface part of which facing the cartilage free cavity, the first surface part of the cartilage membrane carrying a composition comprising at least one stimulation molecule which is capable of inducing a signal transduction in chondroblasts/chondrocytes, introducing, in the cartilage free cavity between the cartilage membrane, the cartilage and the interface, a chondroblast/chondrocyte suspension, and; joining a portion part of the first surface part of the cartilage membrane to the surrounding articular surface so as to sealingly entrap the chondroblast/chondrocyte suspension in the cartilage free cavity using a sealing portion, thereby allowing the chondroblast/chondrocyte suspension to produce and secrete matrix components characteristic for hyalin.

Additionally, the invention relates to a method for in vivo repair of bone and cartilage defects in joints in mammals, such as in osteoarthritic joints, comprising applying, over a bone free cavity and under a cartilage free cavity of a joint, an interface membrane with a first surface part facing the bone free cavity, the interface membrane first surface part carrying a composition comprising at least one stimulation molecule which is capable of inducing a signal transduction in osteoblast/osteocyte, and the second surface part carrying a composition comprising at least one stimulation molecule which is capable of inducing a signal transduction in chondrobtasts/chondrocytes,
introducing, in the interstice between the interface membrane first surface part and the bone, an osteoblast/osteocyte suspension,
joining a portion part of the first surface part of the interface membrane to the surrounding interface surface so as to sealingly entrap the osteoblast/osteocyte suspension in the bone free cavity using a sealing portion, thereby allowing the osteoblast/osteocyte suspension to produce and secrete matrix components characteristic for bone tissue;
applying, over the cartilage free cavity, a cartilage membrane with a first surface part facing the second surface part of the interface membrane, the first surface part of the cartilage membrane carries a composition comprising at least one stimulation molecule which is capable of inducing a signal transduction in chondroblasts/chondrocytes resulting in the chondroblasts/chondrocytes producing and secreting matrix components which form hyalin cartilage,
introducing, in the cartilage free cavity between the interface membrane, the cartilage membrane and the cartilage, a chondroblast/chondrocyte suspension,
joining a portion part of the cartilage membrane to the surrounding articular surface so as to sealingly entrap the chondroblast/chondrocyte suspension in the cartilage free cavity, thereby allowing the chondroblast/chondrocyte suspension to produce and secrete matrix components which form hyalin.

Furthermore by use of the present invention it is possible to provide a method for in vivo repair of bone and cartilage defects in joints in mammals using arthroscopy, such as in osteoarthritic joints. Such a method may comprise treating an interface membrane with a first sealing portion component, applying, over a bone free cavity and under a cartilage free cavity of a joint, an interface membrane with a first surface part facing the bone free cavity, the interface membrane first surface part carrying a composition comprising at least one stimulation molecule which is capable of inducing a signal transduction in osteoblast/osteocyte, and the second surface part, which carries a composition comprising at least one stimulation molecule which is capable of inducing a signal transduction in chondroblasts/chondrocytes,
introducing, in the interstice between the interface membrane first surface part and the bone, an osteoblast/osteocyte suspension,
joining a portion part of the first surface part of the interface membrane to the surrounding interface surface so as to sealingly entrap the osteoblast/osteocyte suspension in the bone free cavity using a second sealing portion component, thereby allowing the osteoblast/osteocyte suspension to produce and secrete components characteristic for bone tissue;
introducing, in the cartilage free cavity between the interface membrane, and the articular surface, a chondroblast/chondrocyte suspension, thereby allowing the chondroblast/chondrocyte suspension to produce and secrete components characteristic for hyalin.

A method for preparation of chondroblast/chondrocyte or osteocyte/osteoblast suspensions may comprise harvesting mesenchymal and/ or mesenchymal precursor cells from a source such as bone marrow, perichondrium, periosteum, blood, blood vessels or muscle; adding the harvested cells to a cell culture flask comprising at least one growth medium; growing the harvested cells until colony forming units with a cell number size in the ranging order of 10-20.000 cells /clone are formed with fibroblastic phenotype (CFU-f); transferring the CFU-f cells into a new cell culture flask comprising at least one selection medium for differentiation of the CFU-f's into chondroblasts/chondrocytes, osteocytes/osteoblasts or myoblasts/myotubes; and harvesting of the differentiated cells.

### Brief description of the drawings

The present invention is further illustrated with reference to the drawings, wherein
Figure 1 shows one embodiment of the present invention applicable for treatment of cartilage defects. The figure shows the anatomy of normal cartilage and a cartilage defect.
Figure 2 shows another embodiment of the present invention applicable for the treatment of cartilage and bone defects. The figure shows the anatomy of normal cartilage and bone and defects of the cartilage and the bone.

### Detailed description of the invention

The invention relates to a method and materials for in vivo repair of cartilage or bone and cartilage defects in joints in mammals, such as human and horses. The chondroblast/chondrocyte cells and the osteoblast/osteocyte cells used in the present invention are cells obtained from a mammal, such as human, horse, pig or calves, preferably autologous cells.

In the present invention, amino add names are used as defined by the Protein DataBank (PDB) which is based on the IUPAC nomenclature ( IUPAC Nomenclature and Symbolism or Amino Acids and Peptides (residue names), Eur. J. Biochem., 138, 9-37 (1984) together with the corrections in Eur. J. Biochem., 152, 1 (1985). The term "amino acid residue" is intended to indicate an amino acid residue contained in the group of arginine (Arg or R), glycine(Gly or G),aspartic acid (Asp or D), proline(Pro or P), histidine (His or H), serine (Ser or S) and asparagine(Asn or N).

### Definitions

The term "chondroblast/chondrocyte suspension" is intended to mean a suspension containing progenitor cells to chondroblasts/chondrocytes or chondroblasts/chondrocytes, which after application to a defect stimulates said cells by a stimulation molecule as defined hereinafter to differentiate and adhere, resulting in the production and secretion of matrix components characteristic for cartilage tissue. Matrix components such as fibrils of type II collagen, and sulfated proteoglycans. chondrotin-6-sulfate and keratan sulfate for the production of hyalin or hyalin articular cartilage.

The term "osteoblast/osteocyte suspension" is intended to mean a suspension containing progenitor cells to osteoblast/osteocyte or osteoblast/osteocyte which after application to a defect stimulates said cells by a stimulation molecule as defined hereinafter to differentiate and adhere, resulting in the production and secretion of components characteristic for bone tissue.

The term "cartilage membrane" is intended to mean a membrane with the ability to bind chondroblasts/chondrocytes to at least one surface part of the membrane. Preferably, the cartilage membrane is capable of binding a stimulation molecule as defined hereinafter and optionally of a structure allowing the chondroblasts/chondrocytes in the chondroblast/chondrocyte suspension to adhere and invade the entire membrane, but not to pass through the membrane. Examples of suitable membranes are collagen I/III membranes purchased from Geistlich Biomaterials, Wollhusen, Switzerland or from Baxter, Denmark.

The term "interface membrane" is intended to mean a membrane with the ability to bind osteoblasts/osteocytes to a first surface part of the interface membrane and chondroblasts/chondrocytes to the second surface part of the interface membrane. Preferably, the interface membrane is capable of binding a stimulation molecule as defined hereinafter to both the first and the second surface part of the interface membrane. Optionally, the interface membrane has a structure allowing an osteoblast/osteocyte suspension to adhere and invade the first surface part of the interface membrane, but not pass through the interface membrane. Furthermore the interface membrane has a structure allowing a chondroblast/chondrocyte suspension to adhere and invade the second surface part of the interface membrane, but not to pass through the membrane. Examples of suitable membranes are collagen I/III membranes purchased from Geistlich Biomaterials, Wollhusen, Switzerland or from Baxter, Denmark.

The term "stimulation molecule" is intended to mean a peptide and/or protein or a fusion thereof, natural or synthetic or a mixture thereof, capable of inducing a signal transduction in chondroblasts/chondrocytes and osteoblasts/osteocytes. Preferably the stimulation molecule, comprises at least the amino acid residues, Arg-Gly-Asp, also named the RGD motif. It will be understood that any motif or amino acid residue sequence, which has the same or substantially the same induction capability as a stimulation molecule comprising a RGD motif could also be used according to the invention.

The term "sealing portion" is intended to mean a glue capable of binding or sealing the membrane and a surface located outside a defect cartilage and/or defect bone area and entrap a chondroblast/chondrocyte and/or a osteoblastlosteocyte suspension into a cartilage free cavity and/or bone free cavity.

The term "growth medium" is intended to mean a growth medium responsible for transferring the mesenchymal stem cells and /or mesenchymal precursor cells obtained from a mammal into colony forming units with fibroblastic phenotype (CFU-f).

The term "selection medium" is intended to mean a selection medium responsible for transferring the CFU-f into more defined cells such as chondroblasts/chondrocytes, osteoblasts/osteocytes or myobtasts/myocubes.

### Cartilage membrane carrying a stimulation molecule

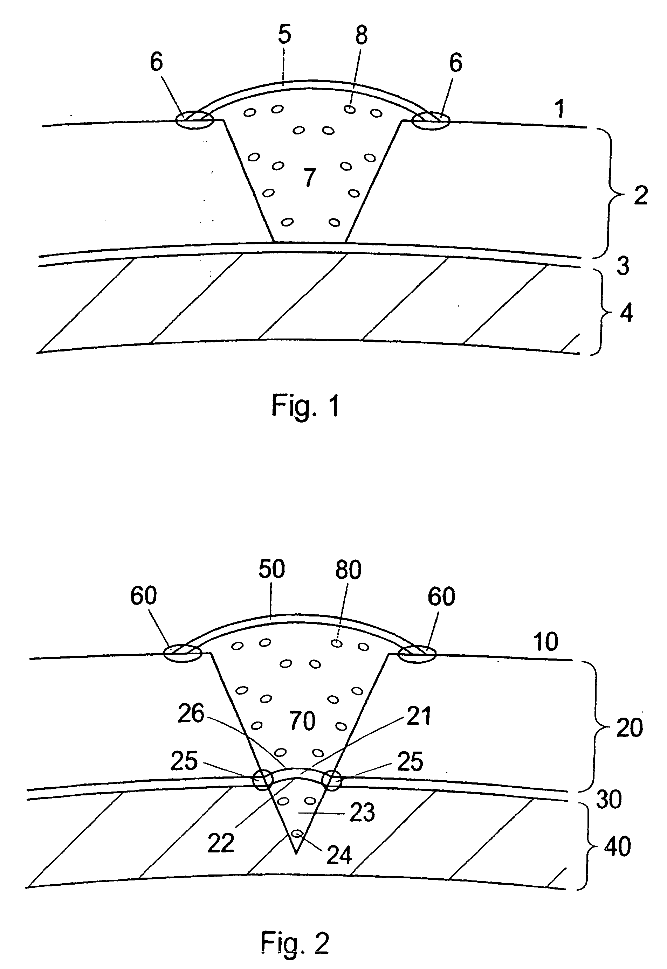

In one aspect, the invention relates to a cartilage membrane carrying a stimulation molecule for use in treatments of cartilage defects in mammals, such as such as chondral leasions or osteochondral lesions, osteochondritis dissecans (OCD), chondromalacia and osteoathritis. Examples of such a cartilage membrane are shown in figure 1 reference 5 and 2 reference 50, respectively. However, the embodiments of the membranes found in figures 1 and 2 are examples of membranes and the invention should not be limited thereto.

The invention relates to a cartilage membrane, having at least one surface part, carrying a composition comprising at least one stimulation molecule, which is capable of inducing a signal transduction in chondroblasts/chondrocytes. Preferably, the cartilage membrane is a non-immunogenic, non-toxic, biodegradable membrane, more preferably, the cartilage membrane is porous or substantially porous, even more preferably, the cartilage membrane has a structure allowing chondroblasts/chondrocytes in a chondroblast/chondrocyte suspension to adhere and invade the entire membrane and most preferably the cartilage membrane is a natural Collagen type I and/or III membrane or a synthetic membrane or part thereof. An example of a cartilage membrane is a periosteal flap excised from the patient's bone, such as for instance the proximal part of the tibia.

The cartilage membrane may be impregnated with the stimulation molecule prior to or directly after application of the membrane to a surface to be treated, such as the cartilage free cavity found in figure 1, reference 7. Preferably the cartilage membrane is impregnated using conventional techniques, such as spraying, painting or immersion or any other conventional technique.

In a preferred embodiment, the stimulation molecule comprises at least one RGD motif. Preferably, the stimulation molecule is a natural or synthetic protein or peptide or a fusion or a mixture thereof, more preferably the stimulation molecule is selected from the group consisting of collagen proteins such as collagen types II, VI, IX, and XI, proteoglycans such as aggregans, decorin, fibromodulin and biglycan, and non collageneous proteins such as cryoprecipitate, fibronectin, vitronectin, fibronogen, fibrillin, kistrin, echistatin, von Willebrand factor, tenascin and anchorin CII, and even more preferably, the stimulation molecule is selected from the group consisting of collagen type II and fibronectin. Additionally, the stimulation molecule is attached to a support.

The final concentration of the stimulation molecule is not critical and is dependent on the degree of the cartilage defect to be treated. The stimulation molecule may be added to a final concentration within the range of nM to mM, preferably between, 1nM to 100 mM and more preferably 10mM.

In another embodiment of the invention the above described cartilage membrane is used for the preparation of a kit containing a cartilage membrane alone or in combination with at least one interface membrane as defined hereinafter for the treatment of cartilage defects or bone and cartilage defects. Furthermore, the kit may comprise other components useful for the treatment of cartilage or bone and cartilage defects in mammals, such as chondral lesions or osteochondral lesions, osteochondritis dissecans (OCD), chondromalacia and osteoathritis.

### Interface membrane carrying a stimulation molecule

In another embodiment, the invention relates to an interface membrane as defined above carrying a stimulation molecule for the treatment of a mammal having a cartilage and bone defect, such as chondral lesions or osteochondreal lesions, osteochondritis dissecans (OCD), chondromalacia and osteoathritis. An example of such an interface membrane is shown in figure 2, reference 21. However, the embodiment of the membrane 21 found in figure 2 is only an example and the invention should not be limited thereto.

The invention relates to an interface membrane with at least two different surface parts, a first and a second surface part, carrying at least one stimulation molecule capable of inducing a signal transduction in chondroblasts/chondrocytes and osteoblasts/osteocytes. The first surface part of the interface membrane has the ability to bind osteoblasts/osteocytes, for example figure 2, reference 22 and the second surface part of the interface membrane has the ability to bind chondroblasts/chondrocytes, for example figure 2, reference 26. Preferably, the interface membrane is a non-immunogenic, non-toxic, biodegradable membrane, more preferably the interface membrane is porous or substantially porous even more preferably the interface membrane has a structure allowing the chondroblasts/chondrocytes in the chondroblast/chondrocyte suspension to adhere and invade one side of the the interface membrane, but not to pass through the interface membrane and even more preferably the interface membrane is a natural or synthetic collagene type I and/or III membrane or part thereof. An example of an interface membrane is a periosteal flap excised from the patient's bone such as for instance the proximal part of the tibia.

The interface membrane may be impregnated with the stimulation molecule prior to or after application of the interface membrane to a surface to be treated, such as for example the bone free cavity found in figure 2, reference 23. Preferably the interface membrane is impregnated using conventional techniques, such as spraying, painting or immersion or any other conventional technique.

In a preferred embodiment, the stimulation molecule comprises at least one RGD motif, preferably, the stimulation molecule is a natural or synthetic protein or peptide or a fusion or a mixture thereof, more preferably the stimulation molecule is selected from the group consisting of collagen proteins such as types II, VI, IX, and XI, proteoglycans such as aggregans, decorin, fibromodulin and biglycan, and non-collageneous proteins such as cryoprecipitate, fibronectin, vitronectin, fibronogen, fibrillin, kistrin, echistatin, von Willebrand factor, tenascin and anchorin CII, and even more preferably, the stimulation molecule is selected from the group consisting of collagen type II and fibronectin. Additionally, the stimulation molecule is attached to a support.

The final concentration of the stimulation molecule is not critical and is dependent on the degree of the defect to be treated. The stimulation molecule may be added to a final concentration within the range of nM to mM, preferably between, 1nM to 100 mM and more preferably 10 mM

According to another embodiment of the invention, the interface membrane may be used for the treatment of bone and cartilage defects using arthroscopy. The interface membrane used for arthroscopy comprises at least one first sealing portion component as defined hereinafter.

Further characteristics of the interface membrane for use in arthroscopy are as defined above for the interface membrane used for treatment of bone and cartilage defects, such as chondral lesions or osteochondreal lesions, osteochondritis dissecans (OCD), chondromalacia and osteoathritis

In another embodiment of the present invention the interface membrane is used for the preparation of a kit containing an interface membrane alone or in combination with at least one cartilage membrane for the treatment of bone defects or bone and cartilage defects. Furthermore, the kit may comprise other components useful for the treatment of cartilage defects or bone and cartilage defects in mammals, such as chondral lesions or ostochondreal lesions, osteochondritis dissecans (OCD), chondromalacia and osteoathritis.

### Method for the propagation of multipotent mesenchymal mammal stem cells to be used for the treatment of cartilage or cartilage and bone defects in mammals.

In the following is described a method for the propagation of multipotent mesenchymal mammal cells and/or precursor cells and their differentiation into chondroblasts/chondrocytes, osteoblasts/osteocytes, fibroblasts, myoblasts/myocytes and adipoblasts/adipocytes without the use of enzymes, such as collagenase. The differentiated cells may be used for the treatment of cartilage and/or bone and cartilage defects in mammals.

The method comprises harvesting mesenchymal and/or precursor cells from a source such as bone marrow, perichondrium, periosteum, blood, blood vessels or muscle;
adding the harvested cells to a cell culture flask comprising at least one growth medium as defined above under the term "growth medium";
growing the harvested cells until colony forming units with a cell number size in the ranging order of 10-20 000 cells /clones are formed with fibroblastic phenotype (CFU-f);
transferring the CFU-f cells into a new cell culture flask comprising at least one selection medium as defined above for further differentiation into chondroblasts/chondrocytes, osteocytes/osteoblasts or myoblasts/myotubes;
harvesting of the differentiated cells and preparation of chondroblast/chondrocyte or osteocyte/osteoblast suspensions.

Preferably, the chondroblast/chondrocyte or osteocyte/osteoblast suspensions may be used for the treatment of cartilage and/or bone and cartilage defects in mammals. The period of cultivation is dependent on the stage and conditions of the harvested mesenchymal and/or precursor cells from mammals and it is therefore difficult to define the time period used for the propagation of the mesenchymal and/or precursor cells in the growth and selection medium to be differentiated into chondroblast/chondrocytes or osteobtasts/osteocytes. The immature cells are primed by selection medium, in which the composition of amino acids, hormonal, growth- and differentiation factors are more specific for selection than for growth. It could be a certain batch of fetal calf serum at a higher concentration than conventionally used (over 10%), up to 30% v/v., which has been tested for its ability to differentiate cells into chondroblasts/chondrocytes, or tested for its ability to differentiate cells into osteohlastslosteocytes. We have internally identified said batches of fetal calf serum that exhibit these selection patterns. Until further a new batch or new batches of fetal calf serum has to be tested for the differentiation of cells. The cells are identified by quantitative PCR of the mRNA (Lane Smith, R., et al., J. Rehab. Research and Development March/April 2000, vol 37), and in case of chondrocyte lines, the cells are tested using monoclonal antibodies such as collagen type II Ab-1 (Clone 582.5) mouse monoclonal antibody (Lab. Vision Corp, Fremont, California).

The use of medium with fetal calf serum identified as sufficient "selection quality" leads to certain differentiations of the precursor cells. Other factors such as physical factors, e.g. adhesion to certain plastic materials or adhesion to cellular or non-cellular adhesion factors also aid the differentiation of the cells.

Preferably the chondroblast/chondrocyte and the osteoblast/osteocyte suspensions contain conventional culture medium such as for example DMEM/F12, serum such as fetal calf serum or autologous serum, preferably a serum, which is non-immunogenic and more preferably the chondroblast/chondrocyte and the osteoblast/osteocyte suspensions comprise chondroblast/chondrocyte respectively osteoblast/osteocyte cells within the range of 100.000 to 10 million cells/ml of the total cell suspensions.

Another method may be a cell cultivating system built on the concept of using the cartilage matrix as the "binding and delivery" (or reservoir) system of growth and differentiation factors to the various cells present in the extracellular matrix, without using enzymatic treated and dissected cartilage pieces. This means, by adding growth factors and differentiation factors though fetal calf serum or separately. Cartilage explants are capable of binding these factors via natural binding proteins present in the matrix. Such a binding and delivery system might increase the half life time of the various growth and differentiation factors present in the culture medium with fetal calf serum, meaning that growth and differentiation factors are much more protected from proteolysis when first bound to natural intermediate matrix molecules (the delivery "vehicles" to the various cell receptors). Additionally, a higher stimulation of the cells by growth and differentiation factors could be speculated, because of a higher local accumulation (higher concentration) of the growth and differentiation factors around the pericellular rim, in the territorial as well as in the interterritorial compartments, compared to the growth and differentiation factors present in conventional medium. A higher local concentration of the growth and differentiation factors, bound to their natural intermediates in the matrix and later "delivered" to the binding receptors (IGF-R, TGF-R, bFGF-R etc.,), induces receptors to cluster formation on the plasma membrane and further signal transduction - growth stimulation. For the growth of the various stimulated cells in the matrix, it is a much more gentle process for the cells to start their propagation and maturation in the natural extracellular matrix, instead of "stripping" off the chondroblast/chondrocytes from their natural matrix components (the cells "history") by the conventional collagenase digestion method. The ability of the chondroblast/chondrocytes to remain viable in cartilage explants in the absence of fetal calf serum or other added growth factors further suggests that the chondrocytes are protected by the extracellular matrix. A tissue culture system with cartilage explants as the starting material for cell propagating, has a number of other advantages over general chondrocyte culture methods. As earlier mentioned, cartilage derived cells in the explants maintain their differentiated state in the matrix while cell stimulation and cell propagation proceed. Second, the cells are not exposed to high concentrations of proteolytic activity used for instance by collagenase digestion of cartilage matrix. This means that the extracellular matrix in the process of propagating the cartilage derived cells is conserved and close to in vivo conditions.

### Sealing portion

The sealing portion may be used to entrap a chondroblast/chondrocyte suspension into a cartilage free cavity and/or an osteoblast/osteocyte suspension into a bone free cavity. Examples of the sealing portions are found in figure 1, reference 6 entrapping the chondroblast/chondrocyte suspension (8) into the cartilage free cavity (7), and the sealing portion in figure 2, reference (25) entrapping the osteoblast/osteocyte suspension (24) into the bone free cavity (23).
An example of a sealing portion is Tisseel™ (Baxter Immuno Austria, a lyophilised virusinactivated substance that consists of fibrinogen, plasmafibrinonectin, factor VIII and plasminogen), which during application is mixed with aprotinin solution, thrombin 4, thrombin 500 and calcium chloride solution, using a dual syringe system connected to one blunt injection needle. Additionally, the "sealing portion'' may comprise a chondroblast/chondrocyte or an osteoblast/osteocyte suspension with or without a stimulation molecule as defined above under the definitions. Another example of a sealing agent is a CoSeal (Cohesion Technologies, Palo Alto, California, U.SA), which also is a two-component biological glue, resembling Tisseel, and can be used instead of Tisseel.

Furthermore, when the sealing portion is glue comprising two different components, one of the components comprising at least one stimulation molecule as defined above may be applied to cover the complete area of at least one side of the membrane. The other component is added between the sealing portion part of the membrane and the edge of the defected cartilage or bone defect after the membrane is placed onto the cartilage or bone defect.

### Treatment of cartilage defects.

In another aspect, the invention relates to a method and materials for *in vivo* repair of cartilage defects in mammals, such as such as chondral lesions or osteochondreal lesions, osteochondritis dissecans (OCD), chondromalacia and osteoathritis.

Reference is now made to Figure 1.

The cartilage-defect part is normally a surface part where the original cartilage has been tom apart. As a preparation for the repair, the defect is debrided so that the cartilage defect surface part appears as a cartilage free cavity 7, often extending down to the interface 3, the interface in between the cartilage 2 and the bone 4, the cartilage-free cavity 7 being surrounded by healthy cartilage 2.

Thereafter, the cartilage membrane 5 as defined above is applied over the cartilage free cavity 7. The cartilage membrane 5 is normally adapted in size so that the portion part of the membrane can be sutured to the surrounding articular surface 1. After application and suturing of the cartilage membrane 5, the edge of the membrane is normally sealed to the surrounding articular surface 1 by means of suitable sealing portions 6. The sealing is not completed; a small part of the circumference is left unsealed to allow the implantation of a chondroblast/chondrocyte suspension 8. Thereafter the cartilage membrane 5 is completely sealed by means of a suitable sealing portion 6.

The cartilage membrane 5, having at least one surface part, carrying a composition comprising at least one stimulation molecule which is capable of inducing a signal transduction in chondroblasts/chondrocytes resulting in the chondroblasts/chondrocytes producing and secreting matrix components which form hyalin or hyalin articular cartilage. The surface part of the cartilage membrane 5 carrying the stimulation molecule is localised against the articular surface 1 and covers the cartilage free cavity 7 comprising the chondroblast/chondrocyte suspension 8 as defined above. Further characteristics of the "cartilage membrane" are defined above under Cartilage membrane carrying a stimulation molecule.

In another embodiment according to the invention a composition is applied to the cartilage defect surface forming the walls surrounding the cartilage free cavity 7 prior to application of the cartilage membrane 5. The composition comprises at least one stimulation molecule as defined above to increase the exposure of the implanted chondroblasts/chondrocytes in the chondroblast/chondrocyte suspension 8 to the stimulation molecule, as well as the capability of the implanted chondroblasts/chondrocytes cells to bind to the surrounding cartilage 2.

Additionally, the chondroblast/chondrocyte suspension 8 is applied simultaneously with a suspension comprising the stimulation molecule as defined above. Preferably, the chondroblast/chondrocyte suspension is a suspension of autologous chondrocytes/chondroblasts. Alternatively, the chondrocyte/chondroblast suspension 8 is applied alone.

In another embodiment according to the invention the chondroblasts/chondrocytes are included in a composition carried by the cartilage membrane 5 and optionally applied directly onto the cartilage free cavity 7 in order to obtain a lining of cells resulting in a normal cartilage surface layer and transitional layer.

Preferably the chondroblast/chondrocyte suspension" contains conventional culture medium such as for example DMEM/F12, serum such as fetal calf serum or autologous serum, preferably a serum which is non-immunogenic and more preferably the chondroblast/chondrocyte suspension comprises chondroblast/chondrocyte cells within the range of 100.000 to 10 millions of cells/ml of the total chondroblast/chondrocyte suspension.

Examples of conditions resulting in cartilage defects, which may be treated according to the invention are chondreal lesions or osteochondral lesions, osteochondritis dissecans (OCD), chondromalacia and osteoathritis.

### Treatment of bone and cartilage defects.

A particular embodiment of the invention relates to method and materials for *in vivo* repair of both bone and cartilage defects in mammals, such as such as chondral lesions or ostochondreal lesions, osteochondritis dissecans (OCD), chondromalacia and osteoathritis using two membranes.
Reference is now made to figure 2.

As a preparation for the repair, the bone and cartilage defect parts is debrided so that the bone and cartilage defect parts appears as bone and cartilage free cavities 23, 70 extending all way through the cartilage 20, interface 30 and down to the bone 40. The bone and cartilage free cavities 23, 70 being surrounded by healthy cartilage 20, interface 30 and bone 40. The interface membrane 21 is applied over the bone free cavity 23 at the level of the interface 30 for the ability to separate the cartilage 70 and the bone 23 free cavities. The interface membrane 21 is applied so that the first surface part 22 of the interface membrane 21, facing the bone free cavity 23 and the second surface part 26 facing the cartilage free cavity 70. The interface membrane 21 is normally adapted in size so that the portion part of the membrane can be sutured to the surrounding area of the interface 30. After application and suturing of the interface membrane 21, the edge of the membrane is normally sealed to the surrounding interface 30 by means of suitable sealing portions 25. The sealing is not completed; a small part of the circumference is left unsealed to allow the implantation of an osteoblast/osteocyte suspension 24. After implantation of the osteoblast/osteocyte suspension 24, the interface membrane is completely sealed to the surrounding interface 30 by means of a suitable sealing portion 25.

The interface membrane 21 has at least two different surface parts, a first 22 and a second 26 surface part, which carries at least one stimulation molecule capable of inducing a signal transduction. The first surface part 22 of the interface membrane 21 has the ability to bind osteoblasts/osteocytes and the second surface part 26, of the interface membrane 21 has the ability to bind chondroblasts/chondrocytes.

Further characteristics of the interface membrane are defined above under Interface membrane carrying a stimulation molecule.

Additionally, the osteobtast/osteocyte suspension is applied simultaneously with a suspension comprising the stimulation molecule as defined above. Preferably the osteoblast/osteocyte suspension is a suspension of autologous osteoblasts/osteocytes. Alternatively, the osteoblast/osteocyte suspension is applied alone.

Preferably the osteoblast/osteocyte suspension contains conventional culture medium such as for example DMEM/F12, serum such as fetal calf serum or autologous serum, preferably a serum which is non-immunogenic and more preferably the osteoblast/osteocyte suspension comprises osteoblast/osteocyte cells within the range of 100.000 to 10 millions of cells/ml of the total osteoblast/osteocyte suspension.

In another embodiment of the invention the osteoblasts/osteocytes in the osteoblast/osteocyte suspension 24 are included in the composition carried by the surface part 22 of the interface membrane 21 and optionally applied directly on the bone free cavity 23 in order to obtain a lining of cells resulting in a normal bone surface layer and transitional layer.

Thereafter, the cartilage membrane 50 as defined above is applied over the cartilage free cavity 70. The cartilage membrane 50 is normally adapted in size so that the portion part of the membrane can be sutured to the surrounding articular surface 10. After application and suturing of the cartilage membrane 50, the edge of the membrane is normally sealed to the surrounding articular surface 10 by means of suitable sealing portions 60. The sealing is not completed; a small part of the circumference is left unsealed to allow the implantation of a chondroblast/chondrocyte suspension 80. Thereafter the implantation is completely sealed to the surrounding articular surface 10 by means of a suitable sealing portion 60.

The cartilage membrane 50, has at least one surface part, carrying a composition comprising at least one stimulation molecule which is capable of inducing a signal transduction in chondroblasts/chondrocytes resulting in the chondrobtasts/chondrocytes producing and secreting matrix components which form hyalin or hyalin articular cartilage. The surface part of the cartilage membrane 50 carrying the stimulation molecule is localised against the articular surface 10 and covering the cartilage free cavity 70 comprising the chondroblast/chondrocyte suspension 80 as defined above. Further characteristics of the cartilage membrane are defined above under Cartilage membrane carrying a stimulation molecule.

The chondroblast/chondrocyte suspension may be applied simultaneously with a suspension comprising of the stimulation molecule as defined above. Alternatively, the chondroblast/chondrocyte suspension may be applied alone.

In another embodiment according to the invention the chondroblasts/chondrocytes in the chondroblast/chondrocyte suspension 80 are included in a composition carried by the cartilage membrane 50 and optionally applied directly on the cartilage free cavity 70 in order to obtain a lining of cells resulting in a normal cartilage surface layer and a transitional layer.

Preferably the chondroblast/chondrocyte suspension contains conventional culture medium such as for example DMEM/F12, serum such as fetal calf serum or autologous serum, preferably a serum, which is non-immunogenic and more preferably the chondroblast/chondrocyte suspension comprises chondrobtasts/chondrocytes within the range of 100.000 to 10 millions of cells/ml of the total chondroblast/chondrocyte suspension.

Examples of conditions resulting in bone and cartilage defects, which may be treated according to the invention are chondral lesions or osteochondral lesions, osteochondritis dissecans (OCD), chondromalacia and osteoarthritis.

According to another embodiment of the invention the bone and cartilage defect is treated using arthroscopy using a two component sealing portion, component 1 and 2. An example of a two component sealing portion is Tisseel Duo Quick (Baxter). The interface membrane 21 is pretreated with a sealing portion component 1. for example a thrombin solution which contains: 500 IU thrombin, 50 mg mammal plasma protein, 40 mol calcium chloride, 10 mg sodium chloride, 3 mg glycine, and water for injection to 1 ml, such as Tisseel Duo Quick (Baxter) Additionally the sealing portion may comprise a stimulation molecule.

As a preparation for the repair, the bone and cartilage defect parts are debrided so that the bone and cartilage defect parts appear as a bone and cartilage free cavity 23, 70 extending all way through the cartilage, interface and down to the bone. The bone and cartilage free cavity is surrounded by healthy cartilage 20, interface 30 and bone 40. A sealing portion component 2 is applied to the surrounding edges of the interface area 30. The interface membrane 21 is applied using arthroscopy over the bone free cavity 23 at the level of the interface 30 for the ability to separate the cartilage 70 and the bone 23 free cavities. The interface membrane 21 is applied so that the first surface part 22 of the interface membrane 21, facing the bone free cavity part 23 and the second surface part 26 facing the cartilage free cavity 70. The interface membrane 21 is normally adapted in size so that the portion part of the membrane can be glued to the surrounding area of the interface 30. After application of the interface membrane 21, the sealing portion component 1 and 2 react with each other and from a glue sealing the interface membrane 21 to the surrounding interface area 30. The sealing is not completed; a small part of the circumference is left unsealed to allow the implantation of an osteoblast/osteocyte suspension 24. After implantation of the osteoblast/osteocyte suspension 24 the interface membrane is completely sealed to the surrounding interface 30 by means of a suitable portion 25. Thereafter the chondroblast/chondrocyte suspension 80 is implantated and the articular surface sealed by means of a suitable portion 60.

The principles of the invention are further illustrated in the appended drawings.

The subsections below describe the components to be used in the methods of this invention.

### Materials and Methods

Growth medium; DMEM/F12 containing 20 % fetal calf serum. DMEM/F12 obtained from Life Technologies Inc., Rockville, MD, USA and fetal calf serum from Life Technologies Inc., Rockville, MD, USA.

### EXAMPLE 1

### Chondrocyte suspension used for the treatment of cartilage defects.

Biopsy of healthy looking cartilage (200-500 mg) is obtained through an arthroscope from a minor load bearing area of the joint under aseptic conditions and placed in a sterile flask containing "Transport medium" (DMEM/F12 with 20% fetal calf serum). The Flask containing Transport medium is delivered within 48 hours to the Cell Culturing Laboratory for further processing into cell culture.
The chondrocytes were expanded in tissue culture flasks in a CO₂ incubator (5% CO₂) at 37°C in growth medium for 3 to 6 weeks, and later on maintained in DMEM/F12 with the patient's (mammal) own heat inactivated serum (range 10-20%) for 3 to 10 days. When the chondrocyte culture has been expanded to the amount of cells needed for the repair of a cartilage lesion(s) of a given patient (mammal), the cells are harvested by trypsinization in 0.25% trypsin in 1mM EDTA, washed in medium containing fetal calf serum (10 20%) and centrifuged at 900 x g for 10 minutes at room temperature, and resuspended to cell numbers between 0.5 to 2 x 10⁶ cells per 0.1 ml growth medium (5 to 20 x 10⁶ cells per 1 ml growth medium). The optimum cell count per 0.1 ml growth medium for implantation is around 1 million cells. In general, a cartilage defect has room for 0.1 ml cellsuspension per 1 cm² defect.

The chondrocytes are delivered to the orthopedic surgeon, who, within 24 hours implants the chondrocytes into a cartilage defect. Prior to the implantation the surgeon has either placed a periosteal flap or a suitable biodegradable cover over the defect, sutured and sealed the flap, except for the injection site. The cells are then injected under the cover, and the injection site is sealed using Tisseel Duo Quick (Baxter).

### EXAMPLE 2

### Stimulation molecule

Collagen II was purified from large scale culturing of porcine chondrocytes as well as proteoglycans derived from the same porcine cartilage source. Collagen, II was obtained from large scale culturing of mammalian chondrocytes (preferably species specific to the recipient of the cells), cultured as explants whereby the adequate amount of Collagen II is present.

### EXAMPLE 3

### Membrane to be used for the treatment and use of the membrane

During open knee surgery, a membrane collagen type I/(III) is used, purchased from Geistlich Biomaterials, Wolhusen, Switzerland or from Baxter (Denmark). The membrane is cut into a size, which is somewhat larger than the cartilage defect, so that the membrane can cover the cartilage defect and extend around ½ cm beyond the rim of the defect. The membrane is sutured together with the cartilage rim. The border between the membrane and the cartilage is sealed using Tisseel Due Quick (Baxter). The collagen type I membrane is pretreated with a 1-5 ml collagen type II solution (3-10 mg collagen II/ml (Dep. Material Engineering, Drexel University, Philadelphia, Pennsylvania) for 5-15 minutes at room temperature prior to use for implantation.
a) When performing arthroscopic surgery, the surface of the collagen I(/III) membrane/which is facing the cartilage defect is pretreated with component II;a thrombin solution which contains: 500 IU thrombin, 50 mg mammal plasma protein, 40 mol calcium chloride, 10 mg sodium chloride, 3 mg glycine, and water for injection to 1 ml, as for instance one part of the Tisseel Duo Quick (Baxter).
b) Under arthroscopy using distention of the joint with gases such as for instance CO₂ instead of distention with solutions such as for instance 0.9% sodium chloride solution, the healthy cartilage creating the rim around the defect is coated with component I which is composed of clottable protein, 75-115 mg thereof 70-110 mg fibrinogen, 2-9 mg fibronectin, 10-50 IU factor XIII, 40-120 g, 3000 KIU aprotinin (bovine), 10-20 mg mammal albumin, 15-35 mg glycine, 2-4 mg sodium chloride, 4-8 mg sodium citrate, 0.2-0.4 mg polysorbate 80, 15 mg creatine monohydrate, and water for injection to 1 ml for instance the other composition of Tisseel.

The above described thin layer membrane coated described by "II" is then inserted over the defect (the size of the membrane is somewhat larger in diameter than the defect) and is placed with the coat facing the defect. When the membrane comes into contact with the coated cartilage rim of the defect described as coated with "II", the two types of coating (coating "I" and coating "II", which are the components in Tisseel) will result in a biological glue, thereby attaching the membrane to the defect. The membrane is held in place by inserting small absorbable pins into the periphery of the defect, anchoring the "sealed" membrane to the cartilage in a more solid manner. The chondrocyte suspension is then injected via a needle in one area of the membrane near the rim into the defect, and the membrane hole is sealed, when pulling the needle, with the two component biological glue such as for instance Tisseel Duo Quick as shown below.

In order to perform the above described arthroscopic procedure, we have in this example used a 2 component biological glue, where component "II" is used as coat on the membrane facing the defect and where component "I" is used as coat on the cartilage rim surrounding the defect. Component "II" will react with component "I", but will not react as glue with medium containing the autologous serum component, but as RGD coat towards the medium, and thereby induce adherence and matrix production of the injected chondrocytes.

The Tisseel 2 component glue can also be used as a coat on the membrane part facing the defect, but will be more difficult to handle when inserted. Another alternative is to coat the cartilage rim surrounding the defect with the two component glue. In this case it will also be difficult to handle the placement of the membrane over the defect.

Tisseel Duo Quick consists of
I: clottable protein, 75-115 mg thereof 70-110 mg fibrinogen, 2-9 mg fibronectin, 10-50 IU factor XIII, 40-120 g, 3000 KIU aprotinin (bovine), 10-20 mg mammal albumin, 15-35 mg glycine, 2-4 mg sodium chloride, 4-8 mg sodium citrate, 0.2-0.4 mg polysorbate 80, 15 mg creatine monohydrate, and water for injection to 1 ml.
II: The thrombin solution contains: 500 IU thrombin, 50 mg mammal plasma protein, 40 mol calcium chloride, 10 mg sodium chloride, 3 mg glycine, and water for injection to 1 ml.

### EXAMPLE 4

### Chondrogenesis of cells in Cambium by proteins containing RGD motifs

Two samples of a periosteal flap from pigs were examined with two different culture medium approaches, one being the growth medium, and a so-called "Conditioned Medium", consisting of the growth medium and 1-20% v/v collagen II. This conditioned medium was used as capable of performing transduction. After conditioning of the medium was completed, this Condition Medium was frozen to -20°C until use.

The growth medium was used as the control medium incapable of inducing chondrocyte formation in the cambium layer of a periosteal flap.

Two samples of a periosteal flap, ½ x ½ cm in size obtained from the medial part of the tibia of a pig were placed in two tissue culture dishes. The conditioned medium was thawed, centrifuged at 3000 rpm for 20 minutes and sterile filtered through a 0.8 my filter and subsequently applied to one of the samples of periost. Incubated at 37° C, in a CO₂ incubator for a period of 4 weeks.

A periosteal flap, ½ x ½ cm in size obtained from the medial part of the tibia of the same animal was used for the control experiment. These samples were incubated at 37°C, in a CO₂ incubator for a period of 4 weeks.

The two samples of periosteal flap were fixed by conventional fixing technique in formaldehyde and stained with various routine staining including Safranin, and submitted to microscopic examination.

### EXAMPLE 5

### Differentiation of stem and/or precursor cells

First, aspirate from spongiosa of femur containing both hematopoetic precursor cells and marrow stromal stem and/or precursor cells was plated into cell culture tissue bottles at approximate dilutions. Single and colony forming marrow cells were stimulated 24 hours (primary incubation) with growth medium. After primary incubation, non-adherent cells such as hematopoetic as well as non-hematopoetic cells were gently removed by replacing supernatant with new media.
Adherent cells were further stimulated 1 week with the above growth medium which induced several colony forming units with cell number sizes ranging in the order of 10-20.000 cells/clone and with fibroblastic phenotype (CFU-f).

After proliferation of these cells in growth medium for another week or more (depending on donor material) some cell cultures were transferred the selection media, are more specific for selection than for growth. It could be a certain batch of fetal calf serum at a higher concentration than conventionally used (over 10%), up to 30% v/v., which has been tested for its ability to differentiate cells into chondroblasts/chondrocytes, or tested for its ability to differentiate cells into osteoblasts/osteocytes. We have internally identified said batches of fetal calf serum that exhibit these selection patterns. Until further a new batch or new batches of fetal calf serum has to be tested for the differentiation of cells. The cells are identified by quantitative PCR of the mRNA (Lane Smith, R., et al., J. Rehab. Research and Development March/April 2000, vol 37), and in case of chondrocyte lines, the cells are tested using monoclonal antibodies such as collagen type If Ab-1 (Clone 5B2.5) mouse monoclonal antibody (Lab. Vision Corp, Fremont, California) for further differentiation and "cell maturation" (2-4 weeks in selection media).

Additionally, developed bone cells were shown to produce mineralized matrix, chondroblast/chondrocytes to form characteristic lacunas where matrix was stained metachomatic with dye such as Safranin-O.

## Claims

1. A cartilage membrane having at least one surface part carrying a composition comprising at least one stimulation molecule, which induces a signal transduction in chondroblast/chondrocytes and which is selected from the group consisting of collagen proteins such as collagen types II, VI, IX, and XI, proteoglycans such as aggregans, decorin, fibromodulin and biglycan, and non-collageneous proteins such as cryoprecipitate, fibronectin, vitronectin, fibronogen, fibrillin, kistrin, echistatin, von Willebrand factor, tenascin and anchorin CII.

2. A cartilage membrane according to claim 1, which is a non-immunogenic, non-toxic, biodegradable membrane.

3. A cartilage membrane according to claims 1 or 2, wherein the membrane material is porous or substantially porous.

4. A cartilage membrane according to claim 3, wherein the membrane is a natural or synthetic collagen type I membrane or part thereof.

5. An interface membrane with a first surface part and a second surface part both carrying a composition comprising at least one stimulation molecule which induces a signal transduction in chondroblast/chondrocytes and in osteoblasts/osteocytes and which is selected from the group consisting of collagen proteins such as collagen types II, VI, IX, and XI, proteoglycans such as aggregans, decorin, fibromodulin and biglycan, and non-collageneous proteins such as cryoprecipitate, fibronectin, vitronectin, fibronogen, fibrillin, kistrin, echistatin, von Willebrand factor, tenascin and anchorin CII.

6. An interface membrane according to claim 5, which is a non-immunogenic, non-toxic, biodegradable membrane.

7. An interface membrane according to claims 5 or 6, wherein the membrane material is porous or substantially porous.

8. An interface membrane according to claim 7, wherein the membrane is a natural or synthetic collagen type I membrane or part thereof.

9. A membrane according to any of the claims 1-8, wherein the stimulation-molecule comprises at least one RGD motif.

10. A membrane according to claim 9, wherein the stimulation molecule is a natural or synthetic protein or peptide or a fusion or a mixture thereof.

11. A membrane according to claim 10, wherein the stimulation molecule is selected from the group consisting of collagen type II and fibronectin.

12. A membrane according to claim 11, wherein the stimulation molecule is attached to a support.

13. A kit for cartilage repair comprising at least one cartilage membrane according to any of claims 1-4 and at least one stimulation molecule according to any of claims 1, 9-12.

14. A kit according to claim 13 comprising at least one interface membrane according to claims 5-8.

15. Use of at least one membrane according to claims 1-12 for the preparation of a kit according to claims 13 or 14 for the treatment of a mammal having a cartilage defects or bone and cartilage defects.

## Patentansprüche

1. Knorpelmembran, die wenigstens einen Oberflächenteil aufweist, der eine Zusammensetzung trägt, die wenigstens ein Stimulationsmolekül umfasst, das in Chondroblasten/Chondrocyten eine Signaltransduktion induziert und das aus der Gruppe ausgewählt ist, die aus Collagen proteinen wie Collagen Typ II, VI, IX und XI, Proteoglycanen, wie Aggreganen, Decorin, Fibromodulin und Biglycan, sowie nichtcollagenartigen Proteinen, wie Kryopräzipitat, Fibronectin, Vitronectin, Fibronogen, Fibrillin, Kistrin, Echistatin, von-Willebrand-Faktor, Tenascin und Anchorin CII, besteht.

2. Knorpelmembran gemäß Anspruch 1, bei der es sich um eine nichtimmunogene, nichttoxische, biologisch abbaubare Membran handelt.

3. Knorpelmembran gemäß Anspruch 1 oder 2, wobei das Membranmaterial, porös oder im Wesentlichen porös ist.

4. Knorpelmembran gemäß Anspruch 3, wobei die Membran eine Membran aus natürlichem oder synthetischem Collagen Typ I oder ein Teil davon ist.

5. Grenzflächenmembran mit einem ersten Oberflächenteil und einem zweiten Oberflächenteil, die beide eine Zusammensetzung tragen, die wenigstens ein Stimulationsmolekül umfasst, das in Chondroblasten/Chondrocyten und in Osteoblasten/Osteocyten eine Signaltransduktion induziert und das aus der Gruppe ausgewählt ist, die aus Collagenproteinen wie Collagen Typ II, VI, IX und XI, Proteoglycanen, wie Aggreganen, Decorin, Fibromodulin und Biglycan, sowie nichtcollagenartigen Proteinen, wie Kryopräzipitat, Fibronectin, Vitronectin, Fibronogen, Fibrillin, Kistrin, Echistatin, von-Willebrand-Faktor, Tenascin und Anchorin CII, besteht.

6. Grenzfiächenmembran gemäß Anspruch 5, bei der es sich um eine nichtimmunogene, nichttoxische, biologisch abbaubare Membran handelt.

7. Grenzflächenmembran gemäß Anspruch 5 oder 6, wobei das Membranmaterial porös oder im Wesentlichen porös ist.

8. Grenzflächenmembran gemäß Anspruch 7, wobei die Membran eine Membran aus natürlichem oder synthetischem Collagen Typ I oder ein Teil davon ist.

9. Membran gemäß einem der Ansprüche 1 bis 8, wobei das Stimulationsmolekül wenigstens ein RGD-Motiv umfasst.

10. Membran gemäß Anspruch 9, wobei das Stimulationsmolekül ein natürliches oder synthetisches Protein oder Peptid oder eine Fusion oder ein Gemisch davon ist.

11. Membran gemäß Anspruch 10, wobei das Stimulationsmolekül aus der Gruppe ausgewählt ist, die aus Collagen Typ II und Fibronectin besteht.

12. Membran gemäß Anspruch 11, wobei das Stimulationsmolekül an einen Träger gebunden ist.

13. Kit für Knorpelreparatur, der wenigstens eine Knorpelmembran gemäß einem der Ansprüche 1 bis 4 und wenigstens ein Stimulationsmolekül gemäß einem der Ansprüche 1 oder 9 bis 12 umfasst.

14. Kit gemäß Anspruch 13, der wenigstens eine Grenzflächenmembran gemäß den Ansprüchen 5 bis 8 umfasst.

15. Verwendung wenigstens einer Membran gemäß den Ansprüchen 1 bis 12 zur Herstellung eines Kits gemäß den Ansprüchen 13 oder 14 für die Behandlung eines Säugers mit Knorpeldefekten oder Knochen- und Knorpeldefekten.

## Revendications

1. Une membrane de cartilage ayant au moins une partie de surface portant une composition comprenant au moins une molécule de stimulation, qui induit une transduction de signal dans les chondroblastes/chondrocytes et qui est sélectionnée dans le groupe consistant en les protéines du collagène telles que le collagène de type II, VI, IX et XI, les protéoglycanes tel que les agrégants, la décorine, la fibromoduline, et le biglycane, et les protéines non-collagéneuses telles que l'un cryoprecipité, la fibronectine, la vitronectine, le fibrinogène, la fibrilline, la kistrine, l'echistatine, le facteur de von Willebrand, la tenascine et l'anchorine CII.

2. Une membrane de cartilage selon la revendication 1, qui est une membrane biodégradable non immunogénique, non toxique.

3. Une membrane de cartilage selon les revendications 1 ou 2, dans laquelle le matériau de la membrane est poreux ou substantiellement poreux.

4. Une membrane de cartilage selon la revendication 3, dans laquelle la membrane est une membrane naturelle ou synthétique de collagène de type I ou une partie de celle-ci.

5. Une membrane interface avec une première partie de surface et une seconde partie de surface, les deux portant une composition comprenant au moins une molécule de stimulation qui induit une transduction de signal dans les chondroblastes/chondrocytes et dans les ostéoblastes/ostéocytes et qui est sélectionnée dans le groupe consistant en les protéines du collagène telles que le collagène de type II, VI, IX et XI, les protéoglycanes tel que les agrégants, la décorine, la fibromoduline, et le biglycane, et les protéines non-collagéneuses tels que l'un cryoprecipité, la fibronectine, la vitronectine, le fibrinogène, la fibrilline, la kistrine, l'echistatine, le facteur de von Willebrand, la tenascine et l'anchorine CII

6. Une membrane d'interface selon la revendication 5, qui est une membrane biodégradable, non immunogénique, non toxique.

7. Une membrane d'interface selon les revendications 5 ou 6, dans laquelle le matériau de la membrane est poreux ou substantiellement poreux.

8. Une membrane d'interface selon la revendication 7, dans laquelle la membrane est une membrane naturelle ou synthétique de collagène de type I ou une partie de celle-ci.

9. Une membrane selon l'une quelconque des revendications 1 à 8, dans laquelle la molécule de stimulation comprend au moins un motif RGD.

10. Une membrane selon la revendication 9, dans laquelle la molécule de stimulation est une protéine ou un peptide, synthétiques ou naturels, ou une fusion ou un mélange de ceux-ci,.

11. Une membrane selon la revendication 10, dans laquelle la molécule de stimulation est sélectionnée dans le groupe consistant en le collagène de type II et la fibronectine.

12. Une membrane selon la revendication 11, dans laquelle la molécule de stimulation est fixée à un support.

13. Un kit pour la réparation du cartilage comprenant au moins une membrane de cartilage selon l'une quelconque des revendications 1 à 4, et au moins une molécule de stimulation selon l'une quelconque des revendications 1, 9 à 12.

14. Un kit selon la revendication 13, comprenant au moins une membrane d'interface selon les revendications 5 à 8.

15. L'utilisation d'au moins une membrane selon les revendications 1 à 12, pour la préparation d'un kit selon les revendications 13 ou 14 pour le traitement d'un mammifère ayant un défaut cartilagineux ou un défaut osseux et cartilagineux.
